(19) Europäisches Patentamt
European Patent Office
Office européen des brevets

(11) EP 3 238 750 A1

(12) **DEMANDE DE BREVET EUROPEEN**

(43) Date de publication:
**01.11.2017 Bulletin 2017/44**

(51) Int Cl.:
***A61L 27/18*** *(2006.01)* ***A61L 27/44*** *(2006.01)*
***A61F 2/50*** *(2006.01)* ***C08L 63/00*** *(2006.01)*

(21) Numéro de dépôt: **16305501.5**

(22) Date de dépôt: **29.04.2016**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA MD**

(71) Demandeur: **C.O.P.**
**26190 Saint Nazaire en Royans (FR)**

(72) Inventeurs:
- **DENIS, David**
**26000 VALENCE (FR)**
- **BOUTEVIN, Bernard**
**34090 MONTPELLIER (FR)**
- **CAILLOL, Sylvain**
**34090 MONTPELLIER (FR)**
- **AUVERGNE, Rémi**
**34000 MONTPELLIER (FR)**
- **BENYAHYA, Sofia**
**26100 ROMANS SUR ISERE (FR)**

(74) Mandataire: **Casalonga**
**Casalonga & Partners**
**Bayerstraße 71/73**
**80335 München (DE)**

(54) **APPAREILLAGE ORTHOPÉDIQUE À BASE DE FIBRES NATURELLES ET D'UNE RÉSINE POLYÉPOXYDE SANS BISPHÉNOL A, NOTAMMENT BIOSOURCÉE**

(57) L'invention concerne un appareillage orthopédique comprenant une ou plusieurs pièces en composite formé de fibres naturelles et d'une matrice, ladite matrice étant à base d'un ou plusieurs polyépoxydes résultant du mélange d'un ou de plusieurs composés (A) comportant deux ou plus de deux groupes époxys avec un ou plusieurs composés (B) comportant deux ou plus de deux groupes amines, ledit mélange étant exempt de bisphénol A et de ses dérivés.

EP 3 238 750 A1

## Description

La présente invention concerne un appareillage orthopédique comprenant au moins une pièce en composite formé de fibres naturelles et d'une matrice, ladite matrice étant à base de polyépoxyde, et son procédé de fabrication. Elle concerne aussi l'utilisation dans l'appareillage orthopédique d'une matrice polyépoxyde obtenue à partir d'au moins un composé (A) comportant deux ou plus de deux groupes époxy et d'au moins un composé (B) comportant deux ou plus de deux groupes amines, lesdits composés (A) étant différents du bisphénol A et de ses dérivés.

Dans le domaine de l'appareillage orthopédique externe sont utilisées des prothèses et des orthèses.

Une prothèse est un dispositif artificiel qui remplace totalement ou partiellement une partie du corps.

Une orthèse est un appareillage qui compense une fonction absente ou déficitaire, assiste une structure articulaire ou musculaire, et/ou stabilise un segment corporel pendant une phase de réadaptation ou de repos. Elle diffère donc de la prothèse qui remplace un élément manquant.

On trouve plusieurs types d'orthèses comme l'orthèse d'orteil ou orthoplastie, les semelles orthopédiques et les corsets.

Actuellement, les prothèses de membre inférieur ou supérieur et certaines orthèses comportent au moins une pièce généralement fabriquée à partir de matériaux composites formés d'une matrice et de fibres comme les fibres de carbone, de poly(p-phénylènetéréphtalamide) (PPD-T) (kevlar), de verre, de polyéthylène de masse molaire très élevée (UHMPE) (Dyneema), ou de polyamide (Nylon).

Cependant, les fibres de carbone présentent l'inconvénient d'être sensibles aux chocs à cause de la rigidité élevée et le faible allongement à la rupture, tout en étant onéreuses.

En outre, elles sont cytotoxiques. Elles présentent ainsi des risques de toxicité pour les poumons et la peau et la manipulation de ces fibres doit donc se faire avec précaution.

Les résines les plus utilisées pour la fabrication des parties composites de prothèses, constituant la matrice, appartiennent à la famille des résines acryliques à base de poly(méthacrylate de méthyle) (PMMA). Elles sont fabriquées par solubilisation du PMMA dans le monomère méthacrylate de méthyle (MAM) et polymérisation en présence de peroxyde(s) organique(s) comme catalyseur(s).

Par exemple, dans le cas de fabrication d'une emboîture pour prothèse de membre inférieur ou supérieur, on dispose les fibres sur le moulage en plâtre d'un moignon, et on les imprègne par procédé d'infusion avec la résine acrylique.

Cependant, ces résines acryliques et les catalyseurs peroxydes présentent des risques importants lors du stockage (résine inflammable type combustible, durcisseur type comburant) et de la manipulation.

Une fois l'emboîture fabriquée, les résines acryliques étant thermoplastiques, elles peuvent donc être ramollies à des températures relativement basses, par exemple d'environ 80°C, et remodelées à maintes reprises sous l'application de la chaleur. Lors de leur mise en forme finale, il est alors possible de rectifier et d'ajuster la forme de la pièce par thermoformage. Cependant, lors de cette étape et sous l'action de la température, le PMMA commence à se décomposer en libérant le monomère méthacrylate de méthyle qui est un composé inflammable, nocif par inhalation et ingestion, et irritant pour la peau, les yeux et les voies respiratoires, et souvent un délaminage du composite a lieu lors de cette retouche.

Un faible taux d'imprégnation des fibres avec ces résines acryliques est observé, et les performances mécaniques des emboîtures, telles que la résistance aux chocs, sont réduites.

Pour pallier ces problèmes, les orthopédistes utilisent plusieurs couches de fibres, qui servent de support, dans le composite et des quantités importantes de résine, ce qui rend les emboîtures épaisses et lourdes et leur usage limité selon le poids du patient et son activité physique.

En ce qui concerne les orthèses, des résines acryliques et polyépoxydes sont utilisées comme matrice, et imprègnent les fibres. Cette technique permet de fabriquer, par exemple, des lames à restitution d'énergie à la place d'un pied. Ainsi les lames offrent un faible poids et une fiabilité mécaniques accrue.

Les résines polyépoxydes actuelles sont préparées à partir du monomère Bisphénol A (BPA). Grâce à ses deux groupements aromatiques, le BPA confère aux matériaux époxy d'excellentes propriétés mécaniques et thermiques.

Cependant, le BPA est un perturbateur endocrinien capable d'interférer avec nos hormones et produire des effets néfastes même à très faibles doses (ppm), comme l'infertilité, le cancer, le diabète et l'obésité.

Les orthèses, telles que les orthèses de jambes, les lames de releveur et les orthèses de membre supérieur, sont ensuite fabriquées par imprégnation de fibres avec la résine polyépoxyde préparée au préalable, puis par application d'un traitement thermique.

Une autre possibilité pour fabriquer ces pièces d'appareillages orthopédiques consiste à utiliser des fibres imprégnées de résine époxy à base de BPA (appelées pré-imprégnés). Ces matériaux sont stockés à froid avant utilisation. Pour la fabrication de la pièce finale, les pré-imprégnés sont ensuite directement déposés sur un moulage puis traités thermiquement.

Cependant, la pré-imprégnation est une technique coûteuse. En effet :

- le stockage et le transport des pré-imprégnés doivent être maintenus à une température relativement basse, notamment inférieure à 10°C;
- le traitement thermique doit s'effectuer à haute température, par exemple de 80°C à 200°C; et
- il est nécessaire d'utiliser des outillages résistants à la température.

Il existe donc un besoin de produire des appareillages orthopédiques comprenant au moins une pièce en composite formé de fibres et d'une résine particulière, permettant de surmonter les inconvénients mentionnés ci-dessus.

L'invention vise à satisfaire ce besoin en proposant de nouveaux appareillages orthopédiques comprenant au moins une pièce en composite formé de fibres naturelles et d'une matrice comprenant un ou plusieurs polyépoxydes tels que définis ci-dessous, lesdits polyépoxydes étant exempts de bisphénol A et de ses dérivés.

Cette combinaison particulière permet de surmonter les inconvénients décrits ci-dessus, et de mettre en oeuvre des matières renouvelables, notamment biosourcées.

En particulier, lesdits polyépoxydes présentent une bonne compatibilité avec les fibres naturelles et une basse viscosité afin d'atteindre une meilleure qualité d'imprégnation, en termes de perméabilité/capacité du renfort à laisser circuler la résine, mouillabilité, taux volumique de fibres/résine et tension superficielle par la mesure de l'angle de contact.

Un premier objet de la présente invention est donc un appareillage orthopédique comprenant au moins une pièce en composite formé de fibres naturelles et d'une matrice, ladite matrice étant à base d'un ou plusieurs polyépoxydes résultant du mélange d'un ou de plusieurs composés (A) comportant deux ou plus de deux groupes époxys avec un ou plusieurs composés (B) comportant deux ou plus de deux groupes amines, ledit mélange étant exempt de bisphénol A et de ses dérivés.

Ces pièces sont généralement réalisées manuellement. Le temps de travail est adapté pour façonner la résine qui va épouser les formes du moulage.

Cette composition particulière de ces pièces d'appareillages orthopédiques permet d'améliorer les conditions de mise en oeuvre et de travail grâce à l'absence du Bisphénol A, à la non-inflammabilité, la faible odeur, la bonne compatibilité avec les fibres et la facilité d'imprégnation.

En outre, le composite permet d'obtenir un faible poids, une dureté élevée, notamment allant de 75 à 90 Shore D, telle que mesurée avec un duromètre modèle HD Hildebrand 3000, et de bonnes propriétés mécaniques, thermiques et élastiques. De plus, ce composite permet de garantir un grand confort aux patients, notamment grâce à la grande capacité d'absorption de chocs, notamment des fibres de lin.

Plus particulièrement, le composite présente une température de transition vitreuse allant de 50 à 200 °C, mieux de 60 à 180 °C, et encore plus préférentiellement de 80 à 150 °C.

On mesure notamment la température de transition vitreuse sur des échantillons rectangulaires de 50 mm x 16 mm x 3 mm. L'échantillon est réticulé à température ambiante pendant 12 heures suivi d'une post-cuisson à 100° C pendant 1h30.

La température de transition vitreuse est mesurée par essai thermomécanique en imposant un mouvement de torsion sous température. L'angle de torsion varie entre 5 ° et 25 °. Le pallier de température est de 20° à 150° et la rampe est de 3 ° C / min. Le temps de relaxation entre la mesure du couple instantané Ci et du couple relaxé Cr est de 2 s. La Tg est mesurée à la différence maximale entre les deux couples.

Dans un mode de réalisation, le composite présente un module de Young allant de 2000 à 3000 MPa, plus préférentiellement de 2200 à 2800 MPa. Un allongement à la rupture préféré va de 3 à 10 %, mieux de 4 à 8 %.

De préférence, la contrainte maximale en flexion va de 80 à 150 MPa, encore plus préférentiellement de 85 à 120 MPa.

Ces tests mécaniques sont réalisés grâce à une machine de traction dans un essai de flexion trois points selon la norme ISO 178. Ils sont effectués sur des éprouvettes de composite de dimensions 66 mm x 21 mm x 3 mm. La vitesse de sollicitation est de 2 mm/min.

Enfin, une meilleure tenue au vieillissement des appareillages orthopédiques fabriqués est obtenue tant au niveau de l'esthétisme et du confort qu'au niveau des propriétés élastiques et mécaniques en rapport avec l'activité et le poids du patient.

Un autre objet concerne un procédé de fabrication d'un appareillage orthopédique comprenant les étapes suivantes :

(i) application d'un sac en plastique, de préférence en poly(alcool de vinyle), sur un moulage en plâtre ;
(ii) disposition des fibres sur le moulage protégé par ledit sac en plastique ;
(iii) préparation d'une résine par mélange d'au moins un composé (A) comportant deux ou plus de deux groupes époxy et d'au moins un composé (B) comportant deux ou plus de deux groupes amines, le mélange étant exempt de bisphénol A et de ses dérivés, à une température allant de 0 à 150°C, de préférence de 10 à 50°C, sous une

pression atmosphérique ou sous vide correspondant à une pression allant par exemple de 500 à 900 mPa, pendant une durée allant de 5 minutes à 36 heures, de préférence de 10 minutes à 24 heures ;
(iv) imprégnation par infusion des fibres avec la résine préparée à l'étape (iii) ; et
(v) traitement à une température allant de 50 à 200 °C, de préférence de 80 à 150 °C, sous pression atmosphérique, pendant une durée allant de 15 à 150 minutes, de préférence de 30 à 120 minutes.

De préférence, un deuxième sac en plastique, par exemple en poly(alcool de vinyle), est placé au début de l'étape (iv), sur l'ensemble des fibres disposées à l'étape (ii), puis la résine est introduite dans le sac et elle est tirée vers la sortie du moule par application d'un vide partiel, ce qui permet l'imprégnation du support par drainage interne.

L'invention a encore pour objet l'utilisation dans l'appareillage orthopédique, d'une matrice polyépoxyde obtenue à partir d'au moins un composé (A) comportant deux ou plus de deux groupes époxy et d'au moins un composé (B) comportant deux ou plus de deux groupes amines, lesdits composés (A) étant différents du bisphénol A et de ses dérivés, en association avec des fibres naturelles.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine, notamment dans les expressions « compris entre » et « allant de ... à ... ».

Par ailleurs, l'expression « au moins un » utilisée dans la présente description est équivalente à l'expression « un ou plusieurs ».

Selon l'invention, l'appareillage orthopédique comprend une ou plusieurs pièces en composite formé de fibres naturelles et d'une matrice étant à base d'un ou plusieurs polyépoxydes résultant du mélange d'un ou de plusieurs composés (A) comportant deux ou plus de deux groupes époxys et d'un ou plusieurs composés (B) comportant deux ou plus de deux groupes amines, ledit mélange étant exempt de bisphénol A et de ses dérivés.

Par exempt de bisphénol A et de ses dérivés, on entend un mélange comprenant 0 % en poids de bisphénol A et/ou de ses dérivés par rapport au poids total dudit mélange.

Par « dérivés de bisphénol A », on entend, par exemple, le diglycidyléther de Bisphénol A (DGEBA) et le diglycidyléther de Bisphénol F (DGEBF) de formules :

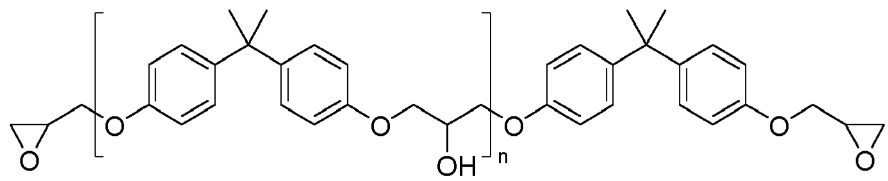
,

et

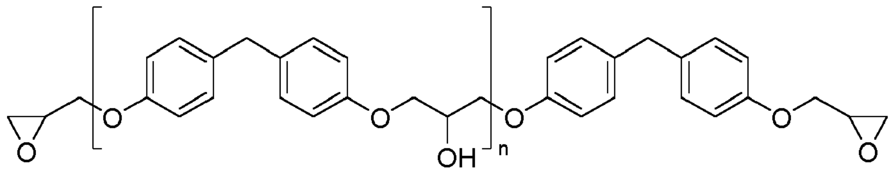

avec n allant de 0 à 1000.

Les fibres naturelles pouvant être utilisées dans l'invention présentent de préférence une masse volumique allant de 1 à 3,5 g/cm$^3$, mieux 1,3 à 3 g/cm$^3$.

Elles sont de préférence des fibres végétales. Elles peuvent être identiques ou différentes.

A titre d'exemples, on peut notamment citer les fibres de lin, de chanvre, de coco, de sisal, de jute, de kénaf et leurs mélanges, mieux les fibres de lin, de chanvre, de coco, de jute et leurs mélanges, et encore mieux les fibres de lin.

En particulier, la fibre de lin est une matière première renouvelable et biodégradable, non-irritante, non-allergène et ne dégage pas de composés organiques volatils (COV).

Les fibres naturelles peuvent se présenter sous la forme de fils, de tissés unidirectionnels, de tissés comprenant des fils dans les directions 0 et 90° ou 45-45°, de non-tissés, de tresses tubulaires ou de rubans.

Le composite contient de 5 à 99 % en poids, de préférence de 40 à 60 % en poids, de fibres naturelles, par rapport au poids total du composite.

Les polyépoxydes utilisés comme matrice dans l'invention sont de préférence partiellement ou totalement

biosourcés. Ils sont préparés à partir de matières premières, à savoir les composés (A) et (B), exemptes de bisphénol A et de ses dérivés.

De préférence, les matières premières utilisées dans la préparation des polyépoxydes selon l'invention, notamment les composés (A) et (B), sont non-fortement toxiques, et mieux biosourcées.

Par « matières premières non fortement toxiques », on entend au sens de la présente invention, des matières premières qui ne sont pas des molécules classées cancérigènes-mutagènes-reprotoxiques (ou CMR), classes 1A, 1B et 2.

Par le terme «biosourcé », on entend au sens de la présente invention un composé issu de la biomasse ayant subi ou non un traitement chimique, et n'étant pas classé CMR, classes 1A, 1B et 2. A titre d'exemples de biomasse, on peut notamment citer les extraits de végétaux, d'arbres, de vigne, de fruits, de légumes ou d'algues.

La réaction entre au moins un composé (A) comportant au moins deux groupes époxy et au moins un composé (B) comportant au moins deux groupes amines peut être représentée par exemple par le schéma suivant, dans le cas où (B) comporte des groupes amines primaires :

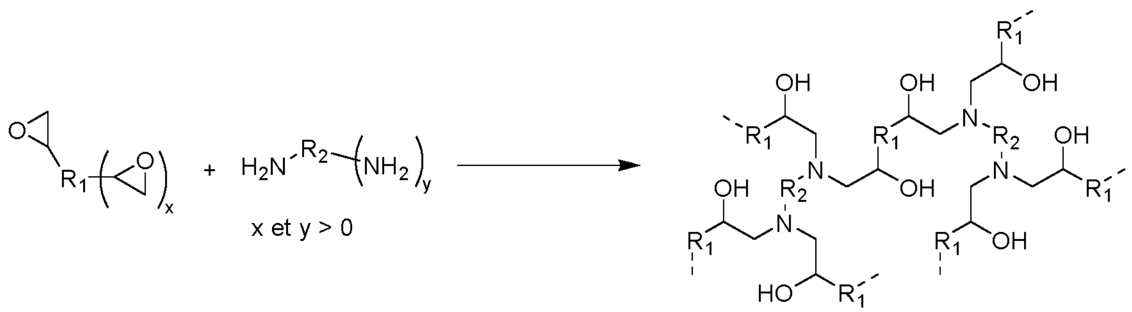


composé (A) composé (B)

dans lequel x varie de 1 à 4 et y varie de 1 à 9, et

$R_1$ et $R_2$ désignent indépendamment l'un de l'autre, une chaîne hydrocarbonée divalente, aliphatique saturée ou insaturée, linéaire ou ramifiée, ou cycloaliphatique ou aromatique, pouvant comporter au moins un hétéroatome tel que O, N et S, et pouvant être substituée.

Les composés (A) comportant deux ou plus de deux groupes époxy et les composés (B) comportant deux ou plus de deux groupes amines pouvant être utilisés, seront décrits plus en détails ci-dessous.

Cette réaction peut être réalisée à une température allant de 0 à 150°C, mieux de 10 à 50 °C, sous une pression atmosphérique ou sous vide.

Cette réaction peut être réalisée en présence d'un catalyseur comme une amine tertiaire telle que la triéthylamine, ou un aminophénol, ou un catalyseur bien connu dans la technique.

L'invention concerne aussi un procédé de fabrication d'une pièce d'appareillage orthopédique selon l'invention. Il comprend les étapes suivantes :

(i) application d'un sac en plastique, de préférence en poly(alcool de vinyle), sur le moulage en plâtre ;
(ii) disposition des fibres sur le moulage protégé par ledit sac en plastique ;
(iii) préparation d'une résine par mélange d'au moins un composé (A) comportant deux ou plus de deux groupes époxy et d'au moins un composé (B) comportant deux ou plus de deux groupes amines, le mélange étant exempt de bisphénol A et de ses dérivés, à une température allant de 0 à 150°C, de préférence de 10 à 50°C, sous une pression atmosphérique ou sous vide correspondant à une pression allant par exemple de 500 à 900 mPa, pendant une durée allant de 5 minutes à 36 heures, de préférence de 10 minutes à 24 heures;
(iv) imprégnation par infusion des fibres avec la résine préparée à l'étape (iii) ; et
(v) traitement à une température allant de 50 à 200 °C, de préférence de 80 à 150 °C, sous pression atmosphérique, pendant une durée allant de 15 à 150 minutes, de préférence de 30 à 120 minutes.

De préférence, en début de l'étape (iv), on place un deuxième sac en plastique, par exemple en poly(alcool de vinyle), sur l'ensemble des fibres disposées à l'étape (ii), puis la résine est introduite dans le sac et elle est tirée vers la sortie du moule par application d'un vide partiel, ce qui permet l'imprégnation du support par drainage interne.

Dans un mode de réalisation, le composé (A) comportant au moins deux groupes époxy peut être préparé à partir d'un polyol comprenant deux ou plus de deux groupes hydroxy, de préférence de 2 à 5 groupes hydroxy et d'épichlorhydrine, d'épibromhydrine ou de glycidol, ou également par la méthode d'allylation et d'oxydation, ces matières

premières étant non-fortement toxiques.

Le procédé selon l'invention peut alors comprendre une étape préalable supplémentaire ($i_0$) consistant à préparer le composé (A) comportant au moins deux groupes époxy à partir d'un polyol comprenant deux ou plus de deux groupes hydroxy, de préférence de 2 à 5 groupes hydroxy et d'épichlorhydrine, d'épibromhydrine ou de glycidol, ou également par la méthode d'allylation et d'oxydation.

La réaction à partir d'un alcool et d'épichlorhydrine, d'épibromhydrine ou de glycidol peut se faire à une température allant de -20 à 150 C, préférentiellement de 0 à 120 °C.

Cette étape ($i_0$) pourra se faire juste avant l'étape (i).

Des exemples de polyols comportant deux ou plus de deux groupes hydroxy pouvant être utilisés dans l'invention sont notamment choisis parmi :

- les alcools aromatiques mononucléiques comme :

  ○ le résorcinol

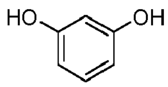

  ○ l'hydroquinone

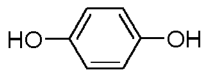

  ○ la vanilline et ses dérivés

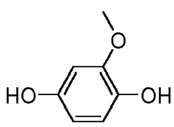 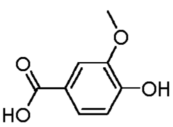 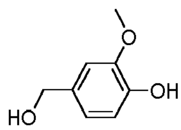

  ○ le phloroglucinol

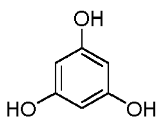

  ○ le cardanol

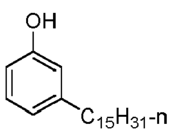 avec n= 0, 2, 4 ou 6

  ○ les dérivés de la lignine choisis parmi l'alcool p-coumarylique, l'alcool coniférylique et l'alcool sinapylique

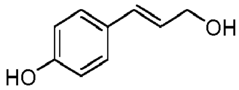
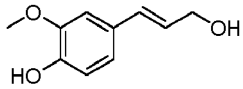
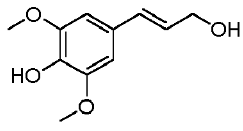

○ les dérivés de la coumarine

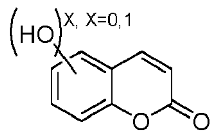

○ l'acide gallique

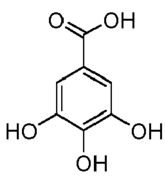 ;

- les alcools aromatiques dinucléiques comme :

○ les dimères de la vanilline

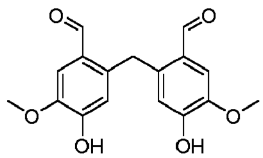

○ les α-ω diphénols

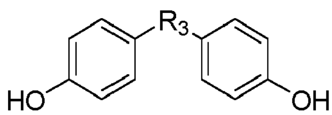

dans laquelle $R_3$ représente une chaîne hydrocarbonée divalente, aliphatique saturée ou insaturée, linéaire ou ramifiée, ou cycloaliphatique ou aromatique, pouvant comporter au moins un hétéroatome tel que O, N et S, et pouvant être substituée, et de préférence représente un groupe alkylène de préférence en $C_{1-20}$, cycloalkylène de préférence en $C_{3-15}$, aromatique de préférence en $C_{6-18}$, pouvant porter des substituants tels qu'alkyle de préférence en $C_{1-5}$comportant éventuellement un ou plusieurs hétéroatomes comme O, N et/ou S ;

- les alcools aromatiques polynucléiques comme :

○ l'oligo-styrol dont le monomère répond à la formule :

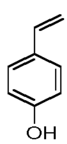

○ le cardanol phénolé

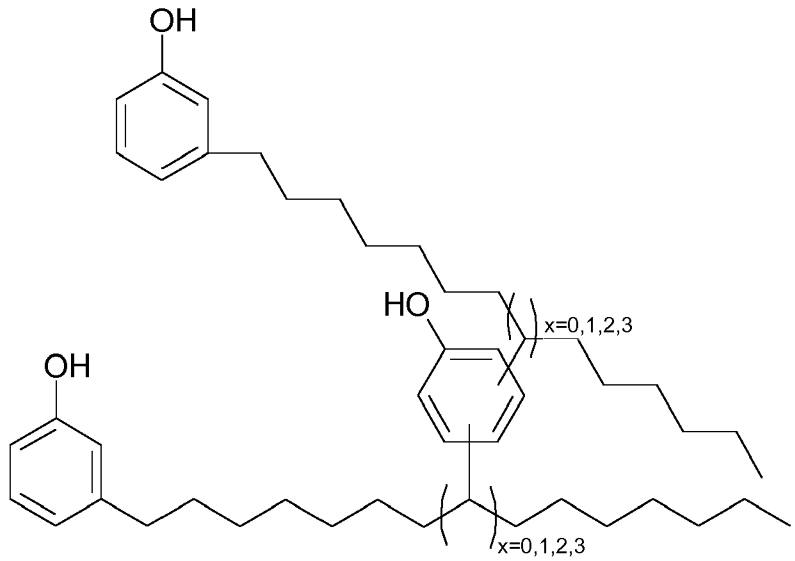

○ les produits de phénolation des huiles et autres polyènes tels que les polybutadiènes (PB), les polyisoprènes (PI), et le farnésène, tels que ceux de formule :

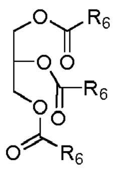

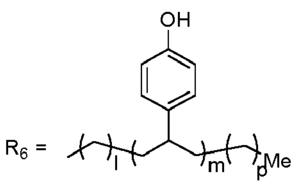

dans laquelle Me désigne le groupe méthyle, 1 est un nombre entier allant de 1 à 10, m est un nombre entier allant de 1 à 3, et p est un nombre entier allant de 1 à 10, sous réserve que 1+3m+p=17 ;

- les alcools aliphatiques comme :

○ les produits obtenus par oxydation des dimères et trimères d'acides gras en $C_{36-54}$, comme :

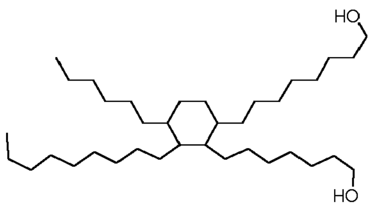

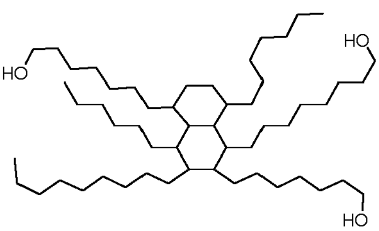

tels que ceux vendus par Croda,

○ les sucres, (oligo)glycérols et isosorbides de formules :

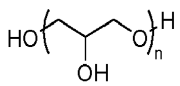

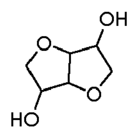

○ les diols, polyéthylène glycols, polypropylène glycols, polybutylène glycols et les oligobutadiènes hydroxy téléchéliques, tels que ceux de formules :

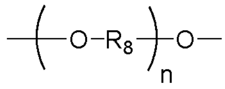

$R_8$ = 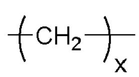 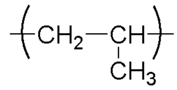

x = 2, 3, 4

avec n allant de 1 à 1000,

○ les polyesters-diols de formule :

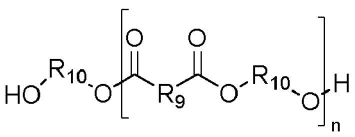

dans laquelle $R_9$ représente un groupe alkylène de préférence en $C_{1-20}$, cycloalkylène de préférence en $C_{3-15}$, aromatique de préférence en $C_{6-18}$, pouvant porter des substituants tels qu'alkyle de préférence en $C_{1-5}$, comportant éventuellement un ou plusieurs hétéroatomes comme O, N et/ou S, et $R_{10}$ est un diol, polyéthylène glycol, polypropylène glycol, polybutylène glycol ou oligobutadiène hydroxy téléchélique tels que ceux définis ci-dessus,

○ les polycarbonates-diols de formule :

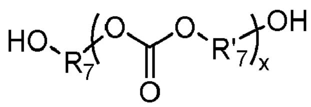

dans laquelle x va de 1 à 1000,

$R_7$ et $R'_7$ représentent chacun, indépendamment l'un de l'autre, un groupe alkylène de préférence en $C_{1-20}$, cycloalkylène de préférence en $C_{3-15}$, aromatique de préférence en $C_{6-18}$, pouvant porter des substituants tels qu'alkyle de préférence en $C_{1-5}$ comportant éventuellement un ou plusieurs hétéroatomes comme O, N et/ou S.

Dans un mode de réalisation, le procédé selon l'invention peut également comprendre une étape préalable supplémentaire ($i'_0$) consistant à préparer le composé (A) comportant au moins deux groupes époxy :

- par époxydation des acides carboxyliques comme l'acide tropique ou encore des acides carboxyliques naturels comme l'acide abiétique :

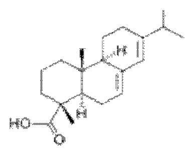

;

- par époxydation des doubles liaisons des composés suivants :

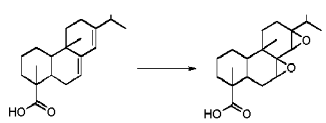

ou le dicyclopentadiène (DCPD) de formule :

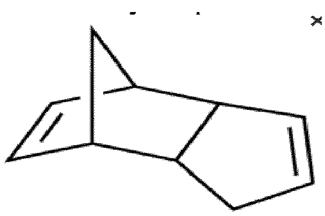

;

ou

- par estérification d'une fonction COOH ; ou
- par transestérification de l'acide éllagique :

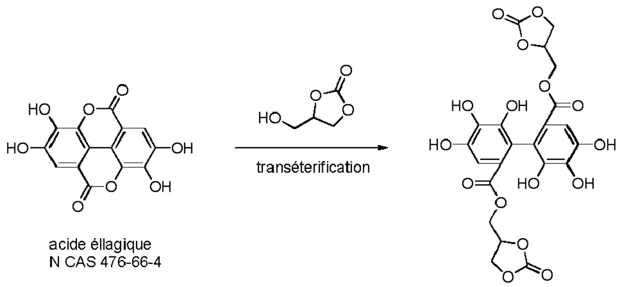


.

Cette étape (i'$_0$) pourra se faire juste avant l'étape (i).

A titre d'exemples de composés (A) contenant deux ou plus de deux groupes époxy, préférés, qui peuvent être commerciaux, on peut notamment citer :

- les dérivés de la vanilline (ou de l'alcool coniférylique) de formules :

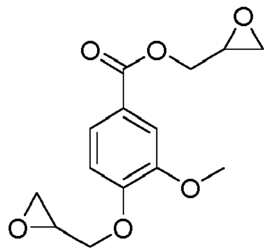
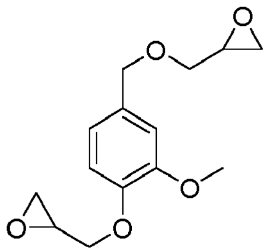
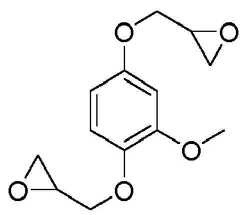

,

- les dérivés de l'alcool para-coumarylique de formules :

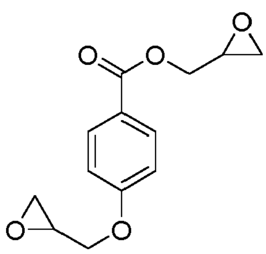
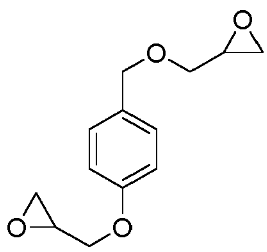
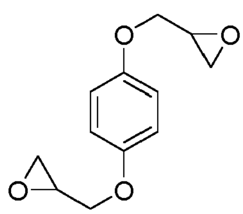

,

- les dérivés de l'alcool sinapylique de formules :

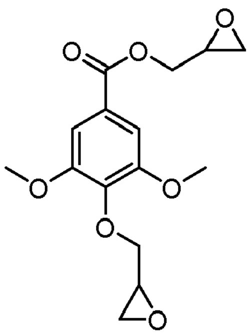
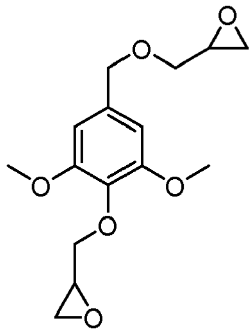
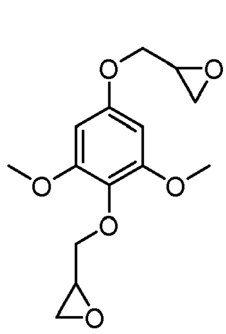
,

- le dérivé de l'acide gallique de formule :

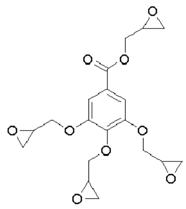
,

- le phloroglucinol époxydé

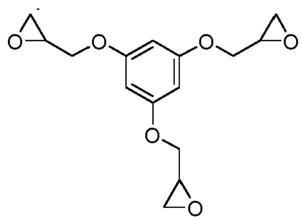
,

- le cardanol polyépoxydé de formule :

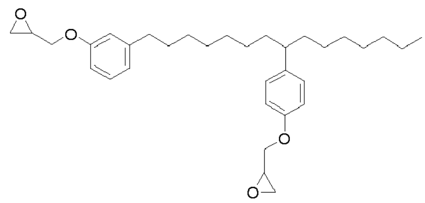
,

tel que celui vendu sous la dénomination commerciale NC-514 par la société Cardolite,

- le diglycidyléther de polypropylène glycol

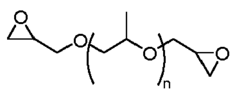

avec n entre 1 et 1000,
tel que celui vendu sous la référence DER® 732P par la société DOW,

- les huiles époxydées et leurs dérivés acides ou esters gras époxydés telles que les huiles de soja époxydées, plus particulièrement celles de formule :

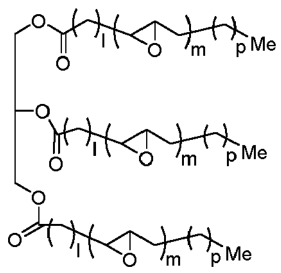

dans laquelle Me désigne le groupe méthyle, 1 est un nombre entier allant de 1 à 10, m est un nombre entier allant de 1 à 3, et p est un nombre entier allant de 1 à 10, sous réserve que 1+3m+p=17. A titre d'exemple de telles huiles, on peut notamment citer celle vendue sous la marque « Vikoflex » par la société Arkema ; et

- les tanins de thé époxydés et la catéchine époxydée.

Plus particulièrement, les composés (A) sont choisis parmi :

- les dérivés de la vanilline (ou de l'alcool coniférylique) de formules :

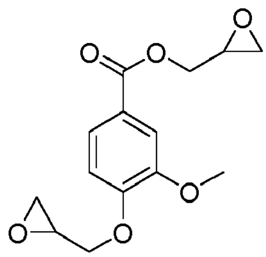 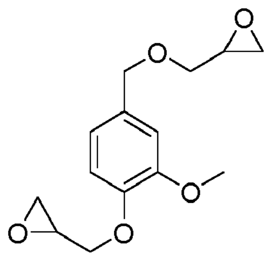 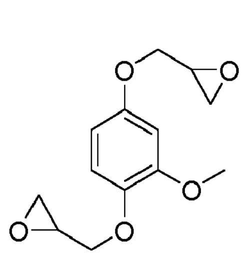

Les composés (B) utilisés dans l'invention comportent deux ou plus de deux groupes amines, de préférence de deux à dix, mieux encore de 2 à 5. Ils peuvent être aromatiques ou aliphatiques.

Les composés (B) comportant deux ou plus de deux groupes amines peuvent être obtenus par une première allylation d'alcools aliphatiques ou aromatiques tels que décrits ci-dessus, ou de crotonisation ou de méthallylation, suivie d'une réaction de thiol-ène sur les doubles liaisons avec un thiol aminé, par exemple, la cystéamine chlorhydratée, en présence d'un amorceur thermique ou photochimique.

L'amorceur thermique peut être de type azoïque comme l'azobisisobutyronitrile (AIBN).

A titre d'exemple d'amorceur photochimique, on peut citer le 2-hydroxy-2-méthyl-1-phényl-propan-1-one telle que celle vendue sous la dénomination commerciale Darocur 1173 ou le bis(2,4,6-triméthylbenzoyl)-phénylphosphine oxide tel que celui vendu sous la dénomination commerciale Irgacure 819.

A titre d'exemples de composés (B) comportant deux ou plus de deux groupes amines, on peut notamment citer :

- les dérivés d'huiles de formule :

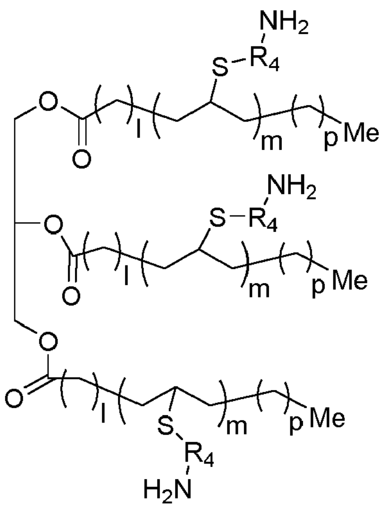

dans laquelle $R_4$ désigne un groupe alkylène en $C_{1-5}$, linéaire ou ramifié, tel que méthylène et éthylène, Me désigne le groupe méthyle, 1 est un nombre entier allant de 1 à 10, m est un nombre entier allant de 1 à 3, et p est un nombre entier allant de 1 à 10, sous réserve que 1+3m+p=17 ;

- les dérivés du polybutadiène portant des groupements $-S-R_4-NH_2$, $R_4$ désignant un groupe alkylène en $C_{1-5}$, linéaire ou ramifié, tel que méthylène et éthylène ;
- les dérivés de l'allylamine de formule :

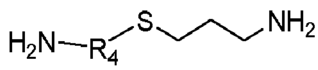

$R_4$ désignant un groupe alkylène en $C_{1-5}$, linéaire ou ramifié, tel que méthylène et éthylène ;

- les dérivés du triméthylolpropane de formule :

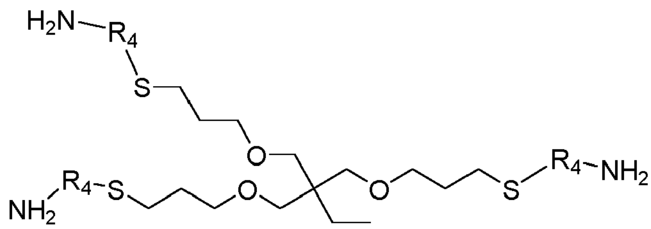

$R_4$ désignant un groupe alkylène en $C_{1-5}$, linéaire ou ramifié, tel que méthylène et éthylène ;

- les dérivés du pentaérythritol de formule :

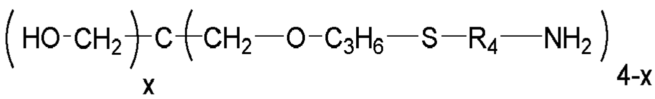

avec 0≤x≤2, et
$R_4$ désignant un groupe alkylène en $C_{1-5}$, linéaire ou ramifié, tel que méthylène et éthylène ;

- les dérivés de cardanol de formules (I) et (II) :

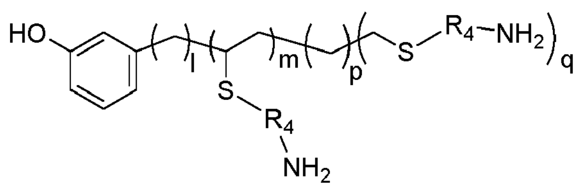

(I)

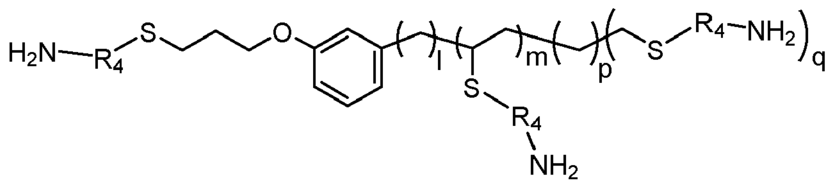

(II)

dans lesquelles $R_4$ désigne un groupe alkylène en $C_{1-5}$, linéaire ou ramifié, tel que méthylène et éthylène, 1 est un nombre entier allant de 1 à 10, m est un nombre entier allant de 1 à 3, p est un nombre entier allant de 0 à 10, et q va de 0 à 1 ;

- les dérivés de vanilline (ou de l'alcool coniférylique) de formules :

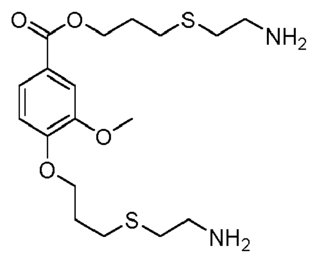

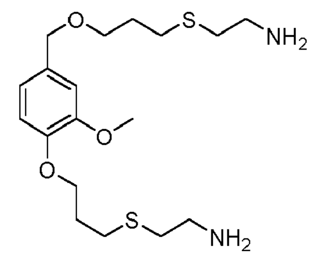

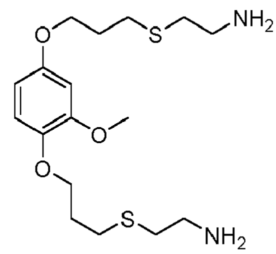

;

- les dérivés de l'alcool paracoumarylique de formules :

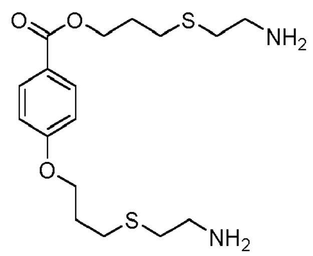

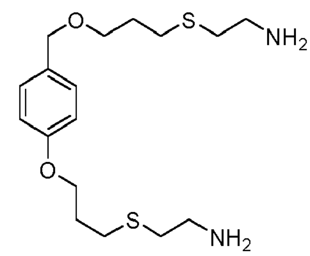

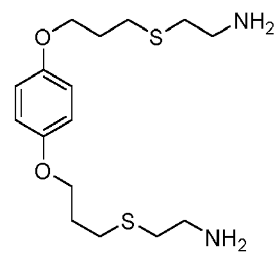

;

- les dérivés de l'alcool sinapylique de formules :

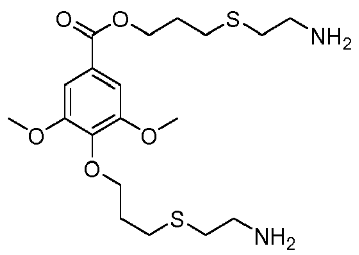

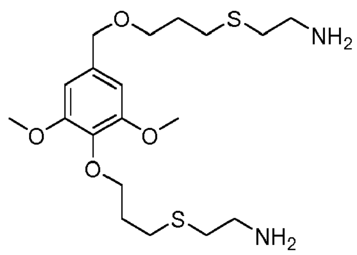

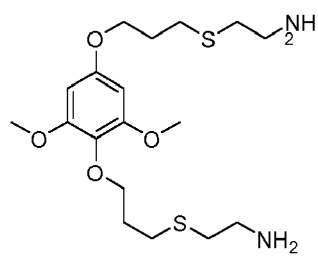

;

- le dérivé d'acide gallique de formule :

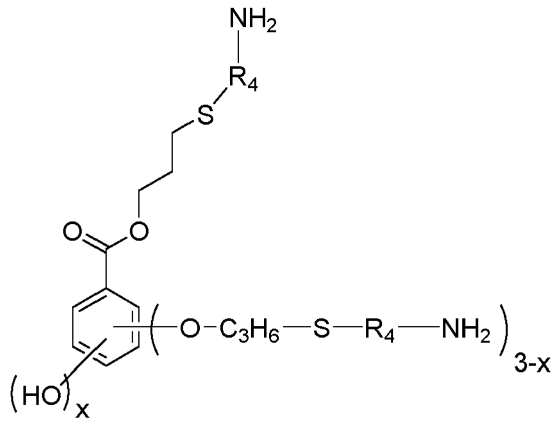

avec 0≤x≤2, et
$R_4$ désignant un groupe alkylène en $C_{1-5}$, linéaire ou ramifié, tel que méthylène et éthylène ;

- les dérivés de tanins, tels que la catéchine de formule :

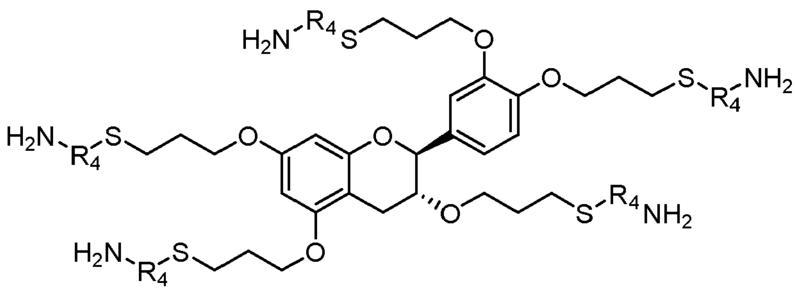

.

$R_4$ désignant un groupe alkylène en $C_{1-5}$, linéaire ou ramifié, tel que méthylène et éthylène.

A titre d'exemples d'autres composés (B) qui peuvent être utilisés dans l'invention et qui sont commerciaux ou qui ont naturellement des composés aminés, on peut notamment citer :

- une phénalkamine de formule :

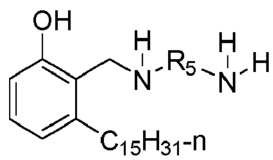

n=0, 2, 4 ou 6
$R_5$:

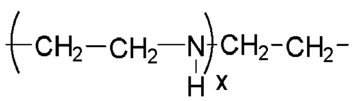

x compris entre 0 et 5

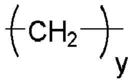

y compris entre 2 et 12

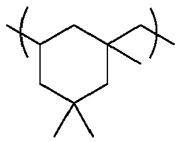

telle que, par exemple, celle vendue sous la dénomination commerciale NX-5454 par la société Cardolite ;

- le dérivé du furane de formule :

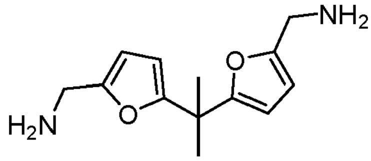

;

- la polylysine ;
- le chitosan et ses oligomères dérivés ;
- les dimères et trimères d'acide gras modifiés en amines et amidoamines comme ceux vendus par la société Croda, de formules :

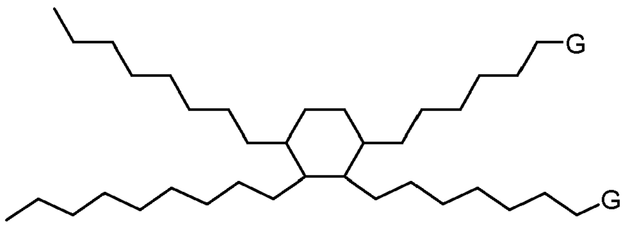

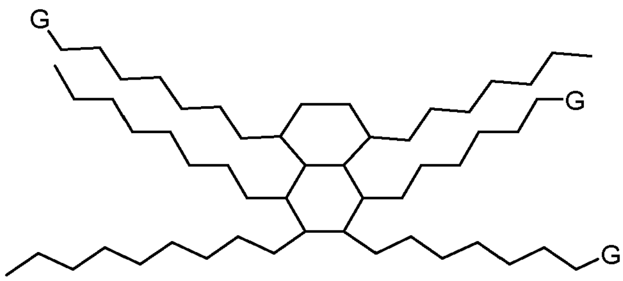

$G=NH_2$;

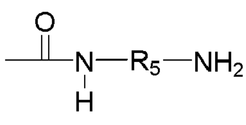

et $R_5$ étant défini comme ci-dessus ;

- les amidoamines comme celles vendues sous la dénomination commerciale Epikure par la société Momentive ;
- les polyéthylènes imines

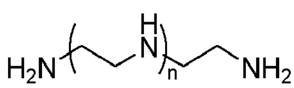

avec n allant de 1 à 10, comme la tétraéthylènepentamine (TEPA, n=3) et la triéthylènetétramine (TETA, n=2) ;

- les polyéther amines telles que celle vendue sous la dénomination commerciale Jeffamine par la société Huntsman

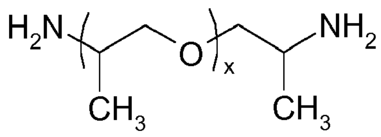

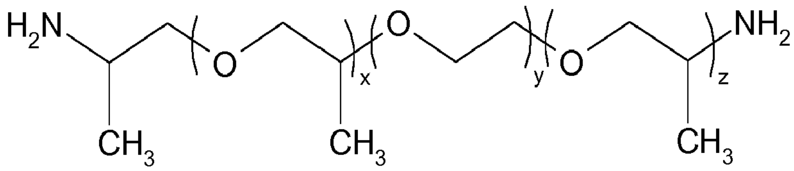

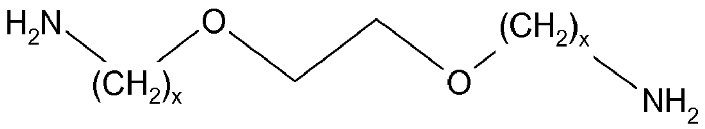

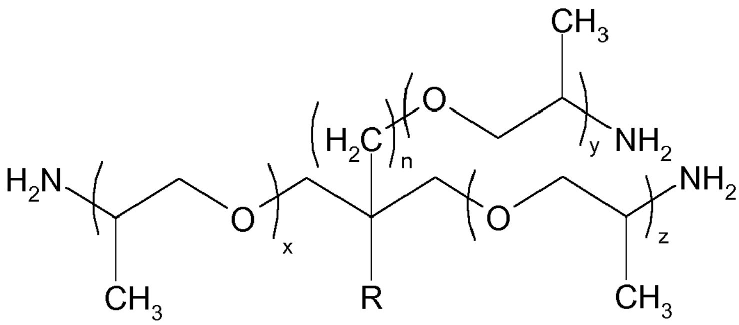

avec x, y, z et n allant de 1 à 1000 ;

- les diamines cycloaliphatiques qui sont des composés dont les fonctions amines sont fixées sur un squelette cyclamique, comme l'isophorone-diamine, la 3,3'-diméthylméthylène-di (cyclohexylamine) (DMDC), la 1,2-diaminocyclohexane-4,4'-méthylènebiscyclohexylamine, et la 4,4'-méthylènebis(2-méthylcyclohexylamine) ; et
- les diamines de formule : 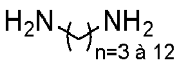 .

Les composés (B) comportant deux ou plus de deux groupes amines préférés sont notamment choisis parmi :

- les dérivés de cardanol de formules (I) et (II) :

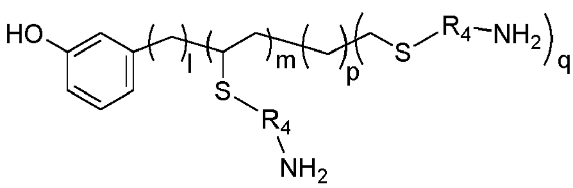

(I)

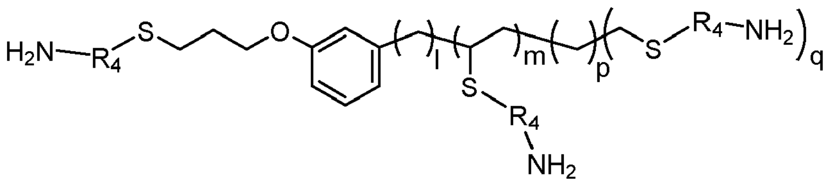

(II)

dans lesquelles $R_4$ désigne un groupe alkylène en $C_{1-5}$, linéaire ou ramifié, tel que méthylène et éthylène, 1 est un nombre entier allant de 1 à 10, m est un nombre entier allant de 1 à 3, p est un nombre entier allant de 0 à 10, et q va de 0 à 1 ;

- les phénalkamines vendues par la société Cardolite, de formule :

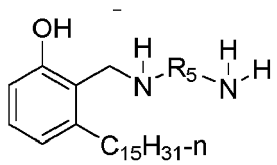

n=0, 2, 4 ou 6
$R_5$:

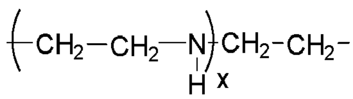

x compris entre 0 et 5

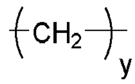

y compris entre 2 et 12

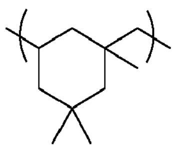

;

- les dimères et trimères d'acide gras modifiés en amines et amidoamines comme ceux vendus par la société Croda, de formules :

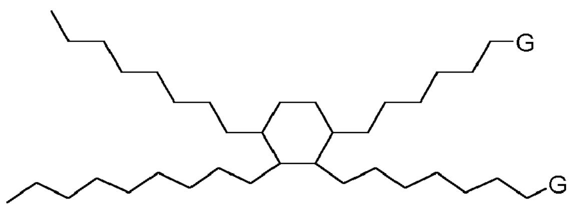

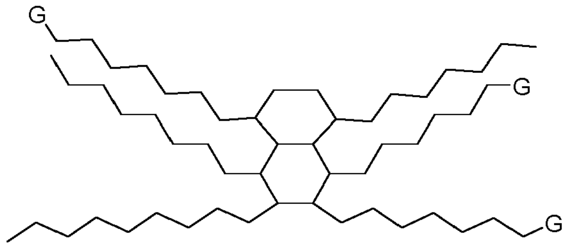

G = $NH_2$ ; 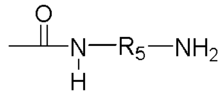

$R_5$ défini comme précédemment

;

et

- les polyéthylènes imines

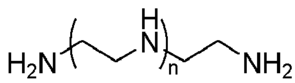

avec n allant de 1 à 10, comme la tétraéthylènepentamine (TEPA, n=3) et la triéthylènetétramine (TETA, n=2) ;

- la polypropylène glycol diamine ou triamine telle que celle vendue sous la dénomination commerciale Jeffamine comme Jeffamine D400 ou la Jeffamine T403 par la société Huntsman, représentée par les formules :

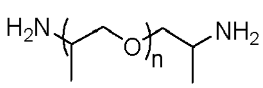 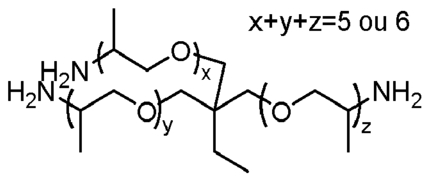

x+y+z=5 ou 6

;

et

- les diamines cycloaliphatiques qui sont des composés dont les fonctions amines sont fixées sur un squelette cyclamique, comme l'isophorone-diamine, la 3,3'-diméthylméthylène-di (cyclohexylamine) (DMDC), la 1,2-diaminocyclohexane-4,4'-méthylènebiscyclohexylamine, et la 4,4'-méthylènebis(2-méthylcyclohexylamine)

Plus particulièrement, les composés (B) sont choisis parmi :

- la polypropylène glycol diamine ou triamine telle que celle vendue sous la dénomination commerciale Jeffamine comme Jeffamine D400 ou la Jeffamine T403 par la société Huntsman, représentée par les formules :

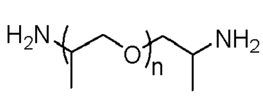 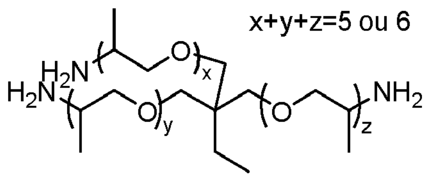

x+y+z=5 ou 6

;

- les diamines cycloaliphatiques qui sont des composés dont les fonctions amines sont fixées sur un squelette cyclamique, comme l'isophorone-diamine, la 3,3'-diméthylméthylène-di (cyclohexylamine) (DMDC), la 1,2-diaminocyclohexane-4,4'-méthylènebiscyclohexylamine, et la 4,4'-méthylènebis(2-méthylcyclohexylamine); et
- les polyéthylènes imines

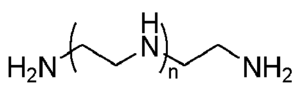

avec n allant de 1 à 10, comme la tétraéthylènepentamine (TEPA, n=3) et la triéthylènetétramine (TETA, n=2).

Le mélange d'un ou de plusieurs composés (A) comportant deux ou plus de deux groupes époxys avec un ou plusieurs composés (B) comportant deux ou plus de deux groupes amines, peut comprendre en outre un monomère (C) de type (méth)acrylate, qui peut être biosourcé.

Ce monomère (C) permet notamment de réduire la viscosité des formulations et de booster la réactivité du système à température ambiante. Il réagit selon l'addition de Michael avec les amines primaires et secondaires, par exemple dans le cas d'un acrylate :

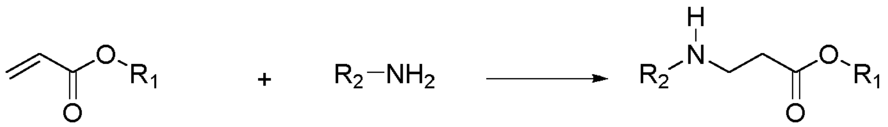

monomère acrylate

adduit acrylate-amine

L'adduit acrylate-amine résultant de cette réaction permet de former très rapidement avec les motifs époxy un réseau polymère très réticulé :

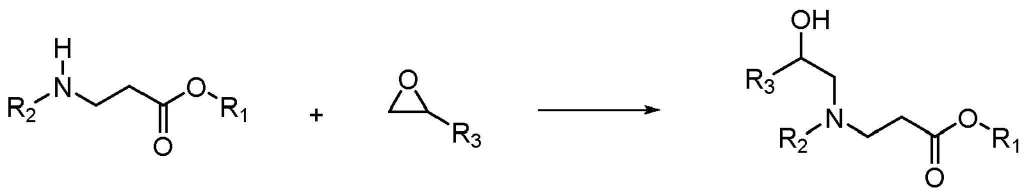

adduit acrylate-amine

époxy

Polymère réticulé

A titre d'exemples de monomères (C) utilisables dans l'invention, on peut notamment citer les méthacrylates et acrylates d'alkyle en $C_{1-8}$, linéaire ou ramifié, et plus particulièrement le méthacrylate de méthyle, d'éthyle ou de n-butyle, l'acrylate d'éthyle, de n-butyle, de t-butyle ou de 2-éthyhexyle ; les méthacrylates et acrylates d'hydroxyalkyle en $C_{1-6}$, par exemple, les méthacrylates de 2-hydroxyéthyle et de 2-hydroxypropyle, les acrylates de hydroxyéthyle, de 2 hydroxypropyle ; les méthacrylates et acrylates d'alcoxyalkyle en $C_{2-10}$, tels que l'acrylate de 2-éthoxyéthyle ; le méthacrylate de tétrahydrofurfuryle, le 2,2-bis[4-(méthacryloxy)phényl]propane, le 2,2-bis[4-(2- méthacryloxyéthoxy)-phényl]propane, le 2,2-bis[4-(2- méthacryloxypropoxy)phényl]-propane ; et les diacrylates et diméthacrylates de polyol comme les diméthacrylates d'éthylène glycol, de diéthylène glycol, de triéthylène glycol, de tétraéthylène glycol et de 1,4-butanediol, et les diacrylates de diéthylène glycol, de triéthylène glycol, de tripropylène glycol, de 1,6-hexanediol et de 1,4-butanediol ; les triacrylates et triméthacrylates de polyol, par exemple, le triacrylate ou triméthacrylate de triméthylolpropane, le triacrylate de pentaérythritol ou le triacrylate de glycérol propoxylé ; l'époxy acrylate ; et leurs produits de réaction entre eux.

De préférence, le mélange des composés (A) et (B), et éventuellement (C) comprend le(s) composé(s) (B) à raison de 1 à 30 % en poids, mieux de 5 à 25 % en poids par rapport au poids total du ou des composés (A).

De préférence, le mélange des composés (A) et (B), et éventuellement (C) comprend le(s) composé(s) (C) à raison de 5 à 20 % en poids par rapport au poids total du ou des composés (A).

La matrice qui comprend un ou plusieurs polyépoxydes résultant du mélange d'un ou de plusieurs composés (A) comportant deux ou plus de deux groupes époxys avec un ou plusieurs composés (B) comportant deux ou plus de deux groupes amines, est présente de préférence en une quantité allant de 1 à 95 % en poids, mieux de 30 à 70 % en poids, et encore mieux de 40 à 60 % en poids par rapport au poids total du composite.

Les fibres naturelles et la matrice polyépoxyde sont de préférence présents dans le composite selon l'invention en un rapport pondéral fibres naturelles sur matrice polyépoxyde allant de 0,05 à 0,99, mieux de 0,4 à 0,6.

Le composite selon l'invention peut comprendre en outre un ou plusieurs additifs ou adjuvants non fortement toxiques, biosourcés ou non, de préférence biosourcés.

Les additifs sont notamment des charges, des renforts et/ou des plastifiants, présents de préférence en une quantité supérieure à 5 % en poids par rapport au poids total de la matrice.

Les adjuvants peuvent être choisis parmi les homopolymères et les copolymères comme des polyacrylates, des polysiloxanes et d'autres vendus par la société BYK, les polymères de cellulose, les latex, les huiles de silicones, les paraffines, les cires, les agents porogènes, les colorants comme les Alumine Trihydrate (ATH) et le dioxyde de titane tels que ceux commercialisés par Huber et Kronos sous le nom Granite Elite et Kronos respectivement, et leurs mélanges.

Ces adjuvants peuvent contribuer à l'homogénéité des constituants de la résine, à l'homogénéité de la structure, à la création de mousses ou à la résistance à l'humidité (hydrophobie). Ces adjuvants sont de préférence présents en une quantité inférieure à 5 % en poids par rapport au poids total de la matrice.

Lorsqu'un ou des additifs et/ou adjuvants est ou sont présent(s), leur quantité peut varier de 0,1 à 20 % en poids, mieux de 0,5 à 10 % en poids par rapport au poids total de la matrice.

Le composite ci-dessus est utilisé pour fabriquer au moins une pièce d'un appareillage orthopédique. A titre d'exemples de pièces d'appareillage orthopédique, on peut citer les emboîtures et la liaison tubulaire de prothèses pour membre inférieur ou supérieur et les orthèses diverses.

Des exemples de tels types d'appareillage orthopédique sont notamment décrits dans les figures annexées.

La Figure 1 est une vue schématique d'une prothèse pour membre inférieur.
La Figure 2 est une vue schématique d'une orthèse releveur du pied.

Dans la Figure 1, la prothèse comprend notamment un manchon souple (1), une emboîture (2), un alignement multiplan (3), une liaison tubulaire (4) et un pied prothétique (5).

Les matériaux possibles pour le manchon (1) sont généralement choisis parmi les copolymères éthylène/acétate de vinyle (EVA), les polypropylènes (PP), les polyéthylènes (PE), les polyuréthanes (PU) et les polyuréthanes thermoplastiques (TPU). Ces matériaux peuvent se présenter sous forme de matériaux souples, matériaux durs ou de mousse.

Le manchon (1) se place sur le moignon (6) et est enfilé dans l'emboîture (2) en matériau composite tel que décrit ci-dessus.

Le manchon (1) sert d'interface entre le moignon (6) et l'emboîture (2). Il assure le confort et amortit les pressions du moignon dans l'emboîture (2).

L'emboîture (2) est la structure rigide de la prothèse qui va supporter le poids du patient et transmettre les mouvements du moignon lors de la marche.

La liaison tubulaire (4) peut être faite à l'aide d'un tube en aluminium ou en composite tel que décrit ci-dessus.

Le pied prothétique (5) est choisi en fonction du niveau d'activité du patient, à savoir marche lente et/ou rapide, piétinement, son activité professionnelle et ses loisirs.

Dans la Figure 2, l'orthèse est un appareillage du segment jambier incluant ou non la zone des métatarsiens destiné à relever le pied en cas de difficulté nerveuse ou musculaire. L'orthèse peut être rigide ou comporter des zones de déformation et d'absorption d'énergie afin de recréer un mouvement le plus naturel et physiologique possible. Elle peut également comporter des articulations.

L'orthèse de la Fig. 2 comprend une embrasse de mollet (20) servant de maintien à l'aide d'une sangle, une lame à restitution d'énergie (21) et une partie antérieure (22) à dureté variable pour un déroulé de pas physiologique. Ladite lame à restitution d'énergie (21) est fabriquée avec le matériau composite tel que décrit ci-dessus.

De préférence, la pièce est une emboîture ou une liaison tubulaire pour prothèse de membre inférieur ou supérieur, ou une orthèse de membre inférieur ou supérieur.

L'invention a encore pour objet l'utilisation dans l'appareillage orthopédique d'un polyépoxyde obtenu à partir d'au moins un composé (A) comportant au moins deux groupes époxy et d'au moins un composé (B) comportant au moins deux groupes amines, comme matrice, en association avec des fibres naturelles.

Le polyépoxyde, les composés (A) et (B) et les fibres naturelles sont tels que décrits ci-dessus.

Tous les autres ingrédients de la matrice polyépoxyde décrits ci-dessus peuvent être utilisés dans l'appareillage orthopédique.

L'exemple suivant est donné à titre purement illustratif de la présente invention.

EXEMPLE

Tout d'abord, on a disposé des fibres de lin présentant une masse volumique de 1,40 g/cm$^3$, sur un moulage en plâtre de moignon, préalablement protégé par un sac en poly(alcool de vinyle). Ce dernier épouse parfaitement le moulage en plâtre.

Une fois les fibres disposées sur le moulage, on a appliqué un autre sac en poly(alcool de vinyle) ouvert sur sa partie supérieure. L'ouverture va permettre d'introduire la résine polyépoxyde.

En parallèle, on a préparé une résine polyépoxyde de la manière suivante. On a mélangé 336,33 g. de vanilline époxydée à un poids équivalent d'époxy (epoxy équivalent weight (EEW)) de 171,09 g/éq. avec 33,67 g. de triacrylate de glycérol propoxylé à température ambiante (18-25 °C). Ensuite, 70,25 g de polyétheramine vendue sous la marque commerciale Jeffamine T403 par la société Huntsman, 45,66 g. d'isophorone diamine vendue sous la marque BD 42 par la société Huntsman et 5,86 g de triéthylènetétramine vendue sous la marque TETA par la société Huntsman ont été ajoutés au mélange précédent. On a également ajouté 24,58 g (5% du mélange époxy/amine) d'un catalyseur vendu par la société CTP sous le nom commercial CETEPOX 1613H, qui est un mélange de 2,4,6-tris(diméthylaminométhyl)phénol et de bis((diméthylamino)méthyl)-phénol.

La résine obtenue a été alors appliquée par infusion en la faisant couler sur les fibres en l'introduisant par l'ouverture existante dans le deuxième sac en poly(alcool de vinyle).

Une fois l'imprégnation des fibres terminée, on a soumis l'ensemble à un traitement thermique à 80 °C pendant 1 h.

On a mesuré les propriétés physiques suivantes avec les méthodes indiquées plus haut :

- Dureté : 85 ShD
- Tg : 120 °C
- Module de Young : 2600 MPa
- Allongement à la rupture : 5,2 %
- Contrainte maximale en flexion : 90,1 MPa

## Revendications

1. Appareillage orthopédique comprenant une ou plusieurs pièces en composite formé de fibres naturelles et d'une matrice, ladite matrice étant à base d'un ou plusieurs polyépoxydes résultant du mélange d'un ou de plusieurs composés (A) comportant deux ou plus de deux groupes époxys avec un ou plusieurs composés (B) comportant deux ou plus de deux groupes amines, ledit mélange étant exempt de bisphénol A et de ses dérivés.

2. Appareillage orthopédique selon la revendication 1, **caractérisé en ce que** les fibres naturelles sont choisies parmi les fibres de lin, de chanvre, de coco, de sisal, de jute, de kénaf et leurs mélanges, de préférence parmi les fibres de lin, de chanvre, de coco, de jute et leurs mélanges.

3. Appareillage orthopédique selon la revendication 1 ou 2, **caractérisé en ce que** le composite contient de 5 à 99 % en poids, de préférence de 40 à 60 % en poids, de fibres, par rapport au poids total du composite.

4. Appareillage orthopédique selon l'une quelconque des revendications précédentes, **caractérisé en ce que** le(s) polyépoxydes sont préparés à partir de matières premières biosourcées.

5. Appareillage orthopédique selon l'une quelconque des revendications précédentes, **caractérisé en ce que** les composés (A) et (B) ne sont pas des molécules classées cancérigènes-mutagènes-reprotoxiques (ou CMR), classes 1A, 1B et 2.

6. Appareillage orthopédique selon l'une quelconque des revendications précédentes, **caractérisé en ce que** les composés (A) contenant deux ou plus de deux groupes époxy sont choisis parmi :

   - les dérivés de la vanilline (ou de l'alcool coniférylique) de formules :

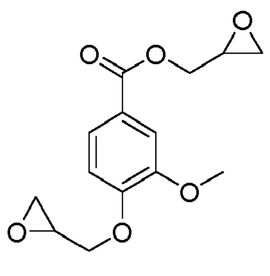
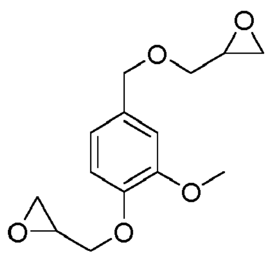
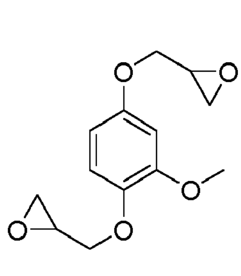
,

   - les dérivés de l'alcool paracoumarylique de formules :

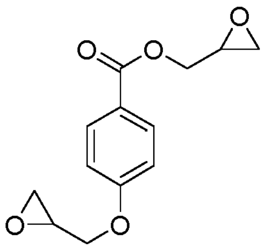 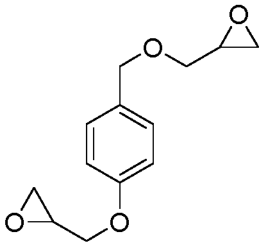 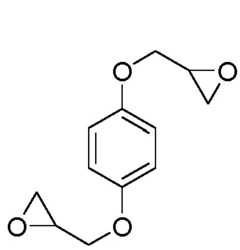 ,

- les dérivés de l'alcool sinapylique de formules :

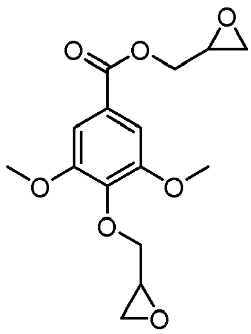 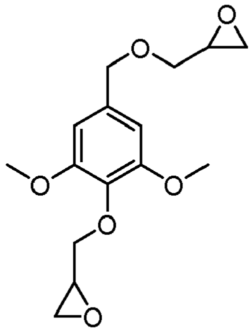 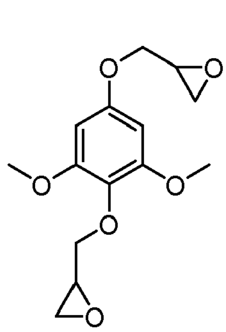 ,

- le dérivé de l'acide gallique de formule :

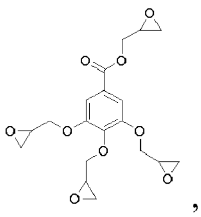 ,

- le phloroglucinol époxydé

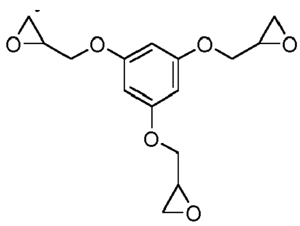 ,

- le cardanol polyépoxydé de formule :

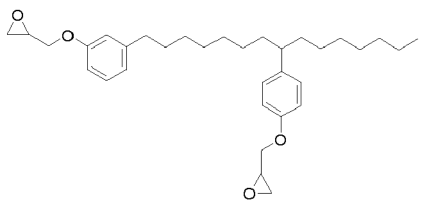 ,

- le diglycidyléther de polypropylène glycol

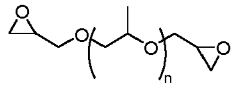

avec n entre 1 et 1000,
- les huiles époxydées et leurs dérivés acides ou esters gras époxydés et
- les tanins de thé époxydés et la catéchine époxydée.

7. Appareillage orthopédique selon l'une quelconque des revendications précédentes, **caractérisé en ce que** les composés (B) comportant deux ou plus de deux groupes amines sont choisis parmi :

- les dérivés d'huiles de formule :

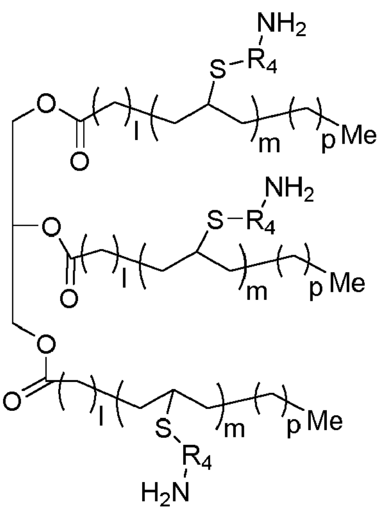

dans laquelle $R_4$ désigne un groupe alkylène en $C_{1-5}$, linéaire ou ramifié, tel que méthylène et éthylène, Me désigne le groupe méthyle, 1 est un nombre entier allant de 1 à 10, m est un nombre entier allant de 1 à 3, et p est un nombre entier allant de 1 à 10, sous réserve que 1+3m+p=17 ;
- les dérivés du polybutadiène portant des groupements $-S-R_4-NH_2$, $R_4$ désignant un groupe alkylène en $C_{1-5}$, linéaire ou ramifié, tel que méthylène et éthylène ;
- les dérivés de l'allylamine de formule :

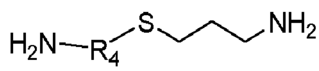

$R_4$ désignant un groupe alkylène en $C_{1-5}$, linéaire ou ramifié, tel que méthylène et éthylène ;
- les dérivés du triméthylolpropane de formule :

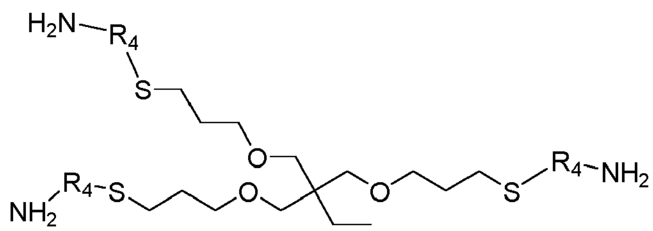

$R_4$ désignant un groupe alkylène en $C_{1-5}$, linéaire ou ramifié, tel que méthylène et éthylène ;
- les dérivés du pentaérythritol de formule :

$$\left(HO-CH_2\right)_x C \left(CH_2-O-C_3H_6-S-R_4-NH_2\right)_{4-x}.$$

avec $0 \leq x \leq 2$, et

$R_4$ désignant un groupe alkylène en $C_{1-5}$, linéaire ou ramifié, tel que méthylène et éthylène ;

- les dérivés de cardanol de formules (I) et (II) :

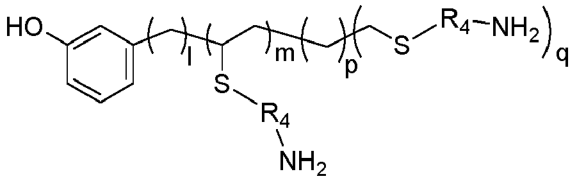

(I)

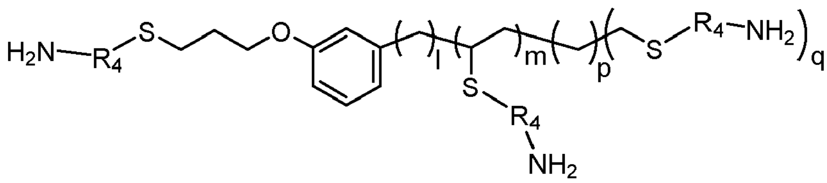

(II)

dans lesquelles $R_4$ désigne un groupe alkylène en $C_{1-5}$, linéaire ou ramifié, tel que méthylène et éthylène, 1 est un nombre entier allant de 1 à 10, m est un nombre entier allant de 1 à 3, p est un nombre entier allant de 0 à 10, et q va de 0 à 1 ;

- les dérivés de vanilline (ou de l'alcool coniférylique) de formules :

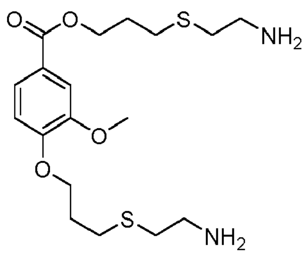

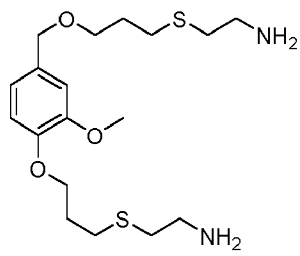

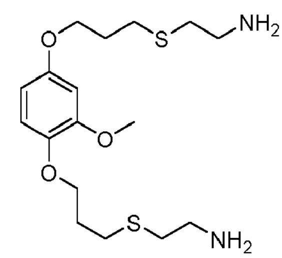

;

- les dérivés de l'alcool paracoumarylique de formules :

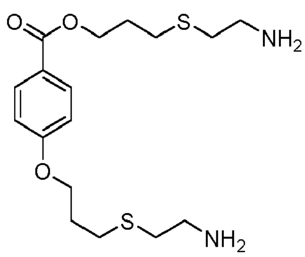

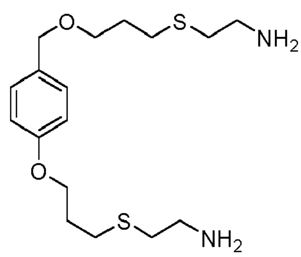

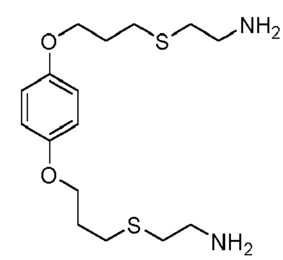

;

- les dérivés de l'alcool sinapylique de formules :

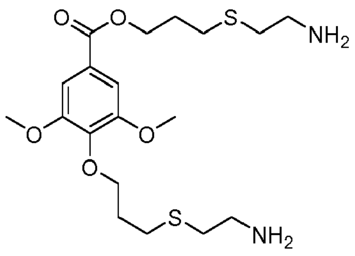

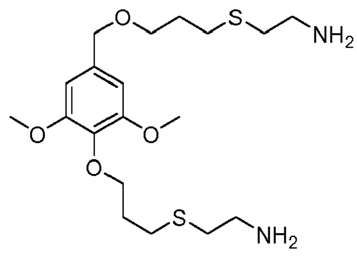

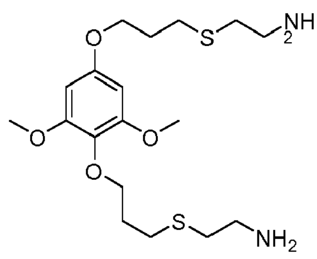
;

- le dérivé d'acide gallique de formule :

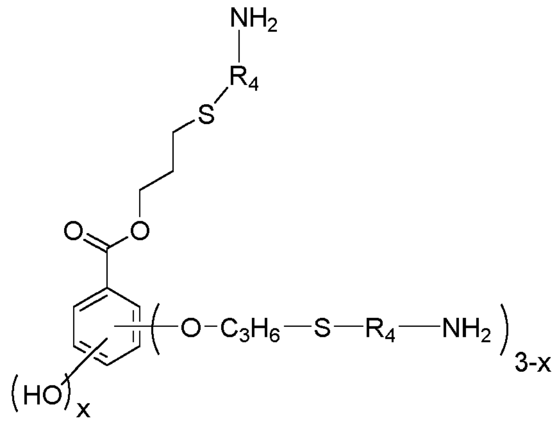

avec $0 \leq x \leq 2$, et

$R_4$ désignant un groupe alkylène en $C_{1-5}$, linéaire ou ramifié, tel que méthylène et éthylène ;

- les dérivés de tanins, tels que la catéchine de formule :

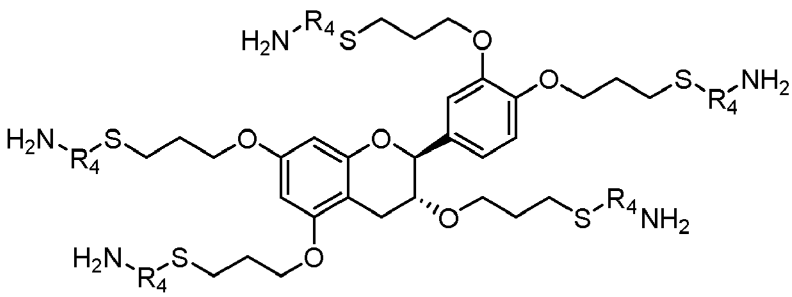

$R_4$ désignant un groupe alkylène en $C_{1-5}$, linéaire ou ramifié, tel que méthylène et éthylène ;

- une phénalkamine de formule :

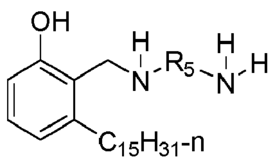

n=0, 2, 4 ou 6

$R_5$:

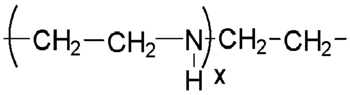

x compris entre 0 et 5

$$\left(-CH_2-\right)_y$$

y compris entre 2 et 12

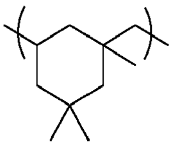

;

- le dérivé du furane de formule :

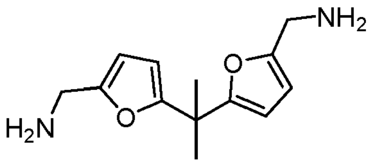

;

- la polylysine ;
- les dérivés du chitosan et leurs oligomères ;
- les dimères et trimères d'acide gras modifiés en amines et amidoamines de formules :

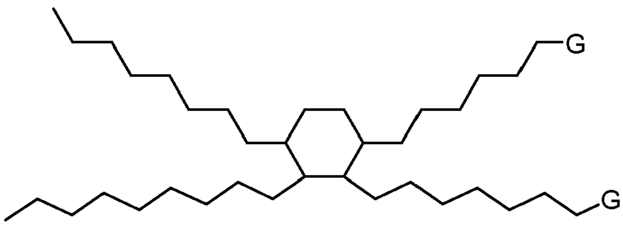

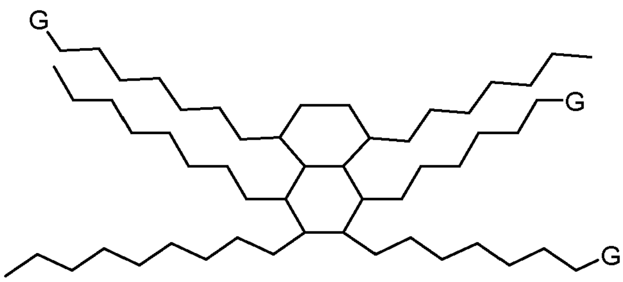

$G=NH_2$;

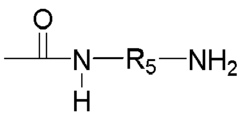

et

$R_5$ désignant $-(CH_2-CH_2-NH)_x-CH_2-CH_2-$ avec x compris entre 0 et 5, $-(CH_2)_y-$ avec y compris entre 2 et 12, ou

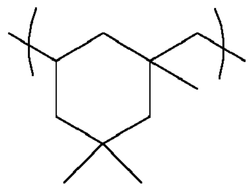

;

- les amidoamines ;
- les polyéthylène-imines

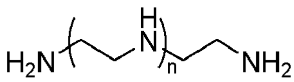

avec n allant de 1 à 10 ;
- les polyéther-amines

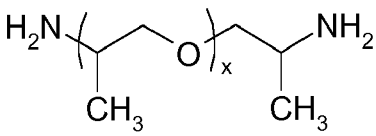

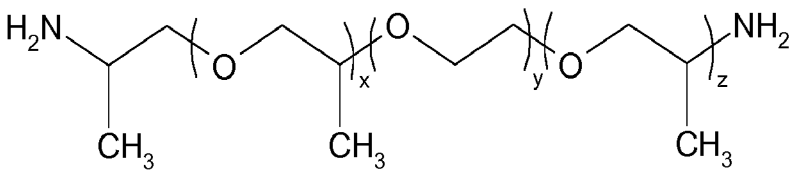

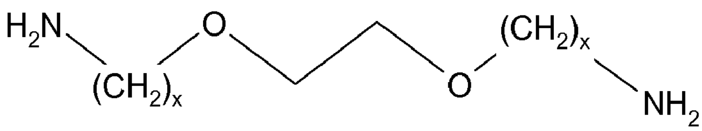

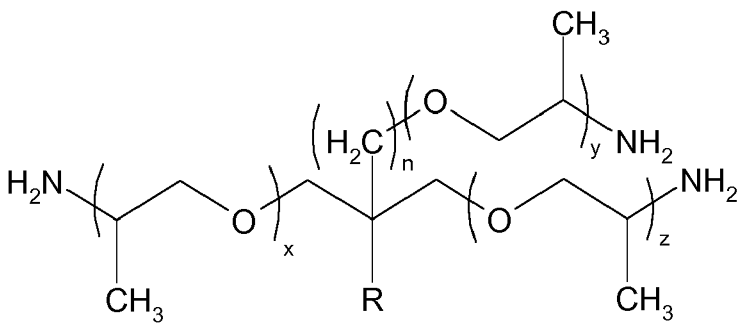

avec x, y, z et n allant de 1 à 1000 ;
- les diamines de formule :

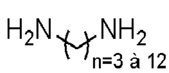

;

et
- les diamines cycloaliphatiques comme l'isophorone-diamine, la 3,3'-diméthylméthylène-di (cyclohexylamine) (DMDC), la 1,2-diaminocyclohexane-4,4'- méthylènebiscyclohexylamine, et la 4,4'-méthylènebis(2-méthylcyclohexylamine);.

**8.** Appareillage orthopédique selon l'une quelconque des revendications précédentes, **caractérisé en ce que** le mélange d'un ou de plusieurs composés (A) comportant deux ou plus de deux groupes époxys avec un ou plusieurs composés (B) comportant deux ou plus de deux groupes amines, comprend en outre un monomère (C) de type (méth)acrylate.

**9.** Appareillage orthopédique selon l'une quelconque des revendications précédentes, **caractérisé en ce que** le composite contient de 5 à 99 % en poids, de préférence de 40 à 60 % en poids, de matrice qui comprend un ou plusieurs polyépoxydes, par rapport au poids total du composite.

**10.** Appareillage orthopédique selon l'une quelconque des revendications précédentes, **caractérisé en ce que** le rapport pondéral fibres naturelles sur matrice qui comprend un ou plusieurs polyépoxydes va de 0,05 à 0,99, mieux de 0,4 à 0,6.

**11.** Appareillage orthopédique selon l'une quelconque des revendications précédentes, **caractérisé en ce que** le composite comprend un ou plusieurs additifs ou adjuvants, de préférence en une quantité allant de 0,1 à 20 % en poids, mieux de 0,5 à 10 % en poids par rapport au poids total de la matrice.

**12.** Appareillage orthopédique selon l'une quelconque des revendications précédentes, **caractérisé en ce qu'**il est une prothèse de membre inférieur ou supérieur ou une orthèse de membre inférieur ou supérieur.

**13.** Appareillage orthopédique selon l'une quelconque des revendications précédentes, **caractérisé en ce que** la pièce est une emboîture ou une liaison tubulaire pour prothèse de membre inférieur ou supérieur.

**14.** Procédé de fabrication d'un appareillage orthopédique selon l'une quelconque des revendications 1 à 13, **caractérisé en ce qu'**il comprend les étapes suivantes :

(i) application d'un sac en plastique, de préférence en poly(alcool de vinyle), sur le moulage en plâtre ;
(ii) disposition des fibres sur le moulage protégé par ledit sac en plastique ;
(iii) préparation d'une résine par mélange d'au moins un composé (A) comportant deux ou plus de deux groupes époxy et d'au moins un composé (B) comportant deux ou plus de deux groupes amines, le mélange étant exempt de bisphénol A et de ses dérivés, à une température allant de 0 à 150°C, de préférence de 10 à 50°C, sous une pression atmosphérique, pendant une durée allant de 5 minutes à 36 heures, de préférence de 10 minutes à 24 heures;
(iv) imprégnation par infusion des fibres avec la résine préparée à l'étape (iii) ; et
(v) traitement à une température allant de 50 à 200 °C, de préférence de 80 à 150 °C, sous pression atmosphérique, pendant une durée allant de 15 à 150 minutes, de préférence de 30 à 120 minutes.

**15.** Utilisation dans un appareillage orthopédique d'une matrice polyépoxyde en association avec des fibres naturelles, ladite matrice résultant du mélange d'un ou de plusieurs composés (A) comportant deux ou plus de deux groupes époxy avec un ou de plusieurs composés (B) comportant deux ou plus de deux groupes amines, comme matrice, lesdits composés (A) étant différents du bisphénol A et de ses dérivés.

FIG.1

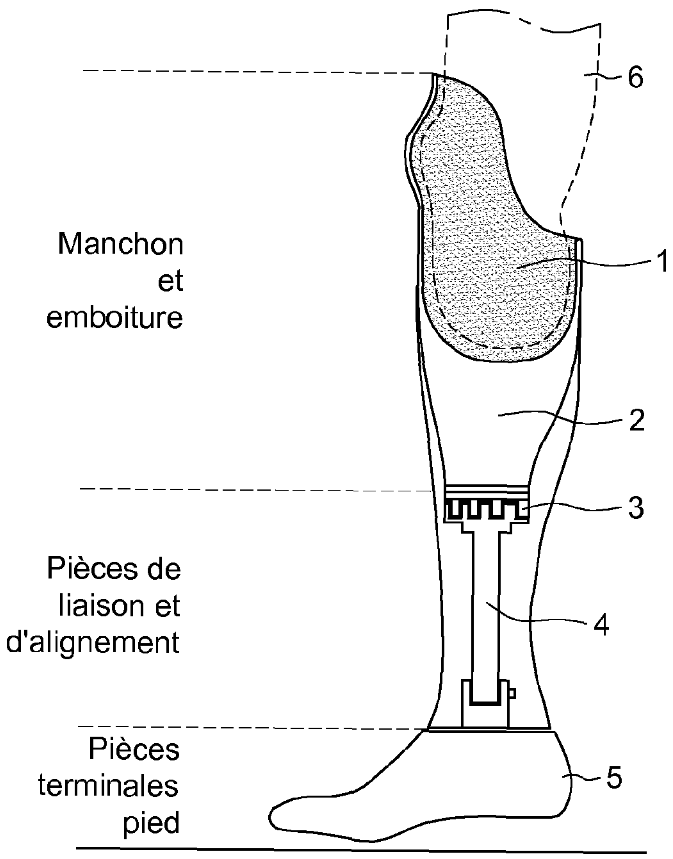
6
Manchon
et
emboiture
1
2
3
Pièces de
liaison et
d'alignement
4
Pièces
terminales
pied
5

FIG.2

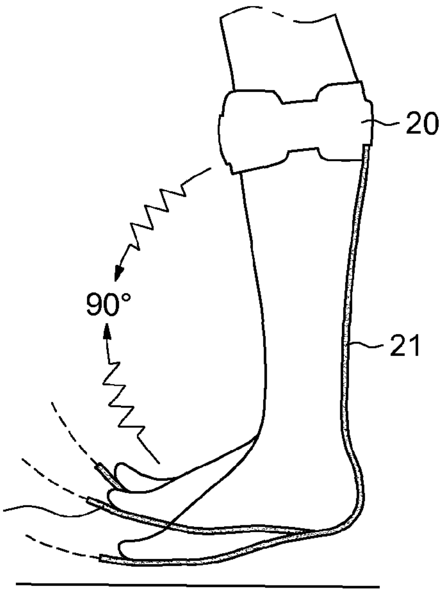
20
90°
21
22

Europäisches Patentamt
European Patent Office
Office européen des brevets

# RAPPORT DE RECHERCHE EUROPEENNE

Numéro de la demande
EP 16 30 5501

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | US 6 514 293 B1 (JANG TAE SEONG [KR] ET AL) 4 février 2003 (2003-02-04) * colonne 4, lignes 62-67; revendications; exemples * | 1-15 | INV. A61L27/18 A61L27/44 A61F2/50 C08L63/00 |
| Y | WO 2015/107114 A1 (DIAM BOUCHAGE [FR]; CENTRE NAT RECH SCIENT [FR]; ECOLE NATIONALE SUPÉR) 23 juillet 2015 (2015-07-23) * page 4 - page 13 * * revendications; exemples * * page 2; revendications; exemples * | 1-15 | |
| A | US 4 822 363 A (PHILLIPS VAN L [US]) 18 avril 1989 (1989-04-18) * le document en entier * | 1-15 | |
| A | US 2008/188948 A1 (FLATT TERRY J [US]) 7 août 2008 (2008-08-07) * le document en entier * | 1-15 | |
| | | | DOMAINES TECHNIQUES RECHERCHES (IPC) |
| | | | A61L A61F C08L |

1

Le présent rapport a été établi pour toutes les revendications

| Lieu de la recherche | Date d'achèvement de la recherche | Examinateur |
|---|---|---|
| La Haye | 20 octobre 2016 | Derrien, Anne-Cécile |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 16 30 5501

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

20-10-2016

| Document brevet cité au rapport de recherche | Date de publication | Membre(s) de la famille de brevet(s) | Date de publication |
|---|---|---|---|
| US 6514293 B1 | 04-02-2003 | DE 10053259 A1 | 31-10-2001 |
| | | JP 2001276102 A | 09-10-2001 |
| | | KR 20010093991 A | 31-10-2001 |
| | | US 6514293 B1 | 04-02-2003 |
| WO 2015107114 A1 | 23-07-2015 | FR 3016368 A1 | 17-07-2015 |
| | | WO 2015107114 A1 | 23-07-2015 |
| US 4822363 A | 18-04-1989 | AU 614510 B2 | 05-09-1991 |
| | | AU 1353188 A | 29-09-1988 |
| | | CA 1299819 C | 05-05-1992 |
| | | CH 675532 A5 | 15-10-1990 |
| | | DE 3808994 A1 | 06-10-1988 |
| | | FR 2612768 A1 | 30-09-1988 |
| | | GB 2202448 A | 28-09-1988 |
| | | GB 2238247 A | 29-05-1991 |
| | | IT 1217363 B | 22-03-1990 |
| | | JP 2928247 B2 | 03-08-1999 |
| | | JP S63277053 A | 15-11-1988 |
| | | SE 468032 B | 26-10-1992 |
| | | US 4822363 A | 18-04-1989 |
| US 2008188948 A1 | 07-08-2008 | AUCUN | |

EPO FORM P0460

Pour tout renseignement concernant cette annexe : voir Journal Officiel de l'Office européen des brevets, No.12/82